Europäisches Patentamt

European Patent Office

Office européen des brevets

(11) Publication number: **0 124 301**
A1

# EUROPEAN PATENT APPLICATION

(21) Application number: **84302307.8**

(22) Date of filing: **04.04.84**

(51) Int. Cl.³: **C 12 N 5/00,** C 12 N 15/00, C 12 N 5/02, A 61 K 39/395

(30) Priority: **06.04.83 US 482538**

(43) Date of publication of application: **07.11.84** Bulletin 84/45

(84) Designated Contracting States: **DE FR GB**

(71) Applicant: **Corning Glass Works, Houghton Park, Corning New York 14831 (US)**

(72) Inventor: **Chen, Pearl Ming-Chu Juan, 12 Knollbrook West Lane, Painted Post New York (US)**
Inventor: **Luderer, Albert August, R.D. 3, P.O. Box 96A Goff Road, Corning New York (US)**
Inventor: **Harvey, Michael Allen, 202 West Hill Road, Painted Post New York (US)**

(74) Representative: **Smith, Sydney et al, Elkington and Fife High Holborn House 52/54 High Holborn, London WC1V 6SH (GB)**

(54) **Human hybrid cell lines, production and use thereof.**

(57) Inter alia a cell line characterised in that it is a substantially pure human myeloma-derived cell line having the ATCC designation CRL-8220 is disclosed, as are cell lines derived therefrom, for example by mutation or hybridization, such as CRL-8221.

Such cell lines may be used in the production of monoclonal antibodies.

The present invention provides advantages over the prior art.

"HUMAN HYBRID CELL LINES, PRODUCTION AND USE THEREOF"

This invention relates to human hybrid cell lines and to the production and use thereof, more particularly, it relates to the production of human hybridomas and to the production of monoclonal antibodies thereby.

In 1975, Kohler and Milstein established procedures for the generation of mouse hybridoma cell lines which secrete monoclonal antibodies, [Nature, 256, 495, (1975)]. Work with mouse hybridoma cell lines has progressed rapidly, at least to some extent because of the availability of inducible mouse plasmacytomas to serve as fusion partners. The readily available mouse myeloma may be induced at will in vivo and proliferates easily in vitro. See, for example, U.S. Patent Nos. 4,172,124; 4,196,265; 4,361,509; 4,361,549 and 4,361,550; Kohler et al., Eur. J. Immunol,, 6, 511, (1976); Milstein et al., Nature, 266, 550, (1977); Koprowski et al., Proc. Natl. Acad. Sci., 74 2985, (1977); Welsh, Nature, 256, 495, (1977), and Melchers et al., Editors, "Lymphocyte Hybridomas. Current Topics in Microbiology and Immunology", Vol. 81, Springer-Verlag, Berlin, 1978.

Much of the work relating to monoclonal antibodies has involved the generation of human-mouse hybridomas; see, e.g., Croce et al., Proc. Natl. Acad. Sci., 76, 3416, (1979); Lane et al., J. Exp. Med., 155, 333, (1982); and Pickering et al., J. Immunol., 128, 406 (1982). A major problem with interspecies hybridomas which secrete human monoclonal antibodies has been a lack of stability. There is a tendency for such hybridomas preferentially to lose human chromosomes. See Croce et al., Eur. J. Immunol., 10, 486, (1980). Intraspecies cell hybrids, as evidenced by mouse-mouse hybridomas, do not share this instability.

Progress toward the development of human-human hybridoma cell lines has been slow, primarily because of the unavailability of a suitable human myeloma cell line. Only one new human myeloma cell line has been reported since 1968; see Karpas et al., Science, 216, 977, (1982).

This does not mean, however, that no work has been carried out with respect to human-human hybridomas. For example, Olsson et al., Proc. Natl. Acad. Sci., 77, 5429, (1980); and Croce et al., Nature, 288, 488, (1980),have reported the production of human hybridomas which secrete human monoclonal antibodies. The human myeloma cells employed were mutants of the human B cell lines U266 and GM1500, respectively; see also GB 2,086,937A. In addition, a human-human hybridoma system based on a fast-growing mutant of the ARH-77 plasma cell leukemia-derived line has been described by Edwards et al., Eur. J. Immunol., 12, 641, (1982).

Thus, there is still a need for a human myeloma cell line capable of fusing with lymphocytes, especially human lymphocytes, to generate with improved frequencies human hybridoma cell lines.

It is therefore an object of the present invention to provide a new, continuously proliferating, or perpetual, human myeloma-derived cell line.

It is another object of the present invention to provide a hypoxanthine phosphoribosyl transferase-deficient mutant of a new, perpetual human myeloma-derived cell line, which mutant is capable of hybridization with lymphocytes.

Still another object of the present invention is to provide an improvement in a process for producing a hybrid cell line from a lymphocyte and a myeloma-derived cell.

A further object of the present invention is to provide a hybrid cell line produced by fusing cells of a human myeloma-derived cell line with lymphocytes sensitized to an immunogen.

Yet a further object of the present invention is to provide a process for the production of monoclonal antibodies by a hybrid cell line.

Yet another object of the present invention is to provide an improvement in a process for the transfection of lymphocytes.

Accordingly, the present invention provides a substantially pure human myeloma-derived cell line which has been deposited with the American Type Culture Collection (ATCC), 12301 Parklawn Drive, Rockville, Maryland, USA having the ATCC designation CRL-8220.

The present invention also provides a daughter line of the human myeloma-derived cell line having the ATCC designation CRL-8220. For the present purposes, a cell line is considered to be a daughter of the cell line having the ATCC designation CRL-8220 if it is derived therefrom, such as by mutation or hybridization.

In one embodiment, a mutant cell line is provided which is capable of hybridization with lymphocytes and which has at least one characteristic which is different from the cell line having the ATCC designation CRL-8220, whereby such mutant cell line will proliferate under conditions which will not sustain the cell line having the ATCC designation CRL-8220. Such mutant cell line may be deficient in hypoxanthine phosphoribosyl transferase; an example of such a cell line is the cell line deposited with the American Type Culture Collection, having the ATCC designation CRL-8221.

In another embodiment, a hybrid cell line is provided which is produced from cells of a mutant cell line by the fusion of such cells with lymphocytes sensitized to an immunogen, which mutant cell line is derived from the cell line having the ATCC designation CRL-8220 and which is capable of hybridization with lymphocytes and which has at least one characteristic which is different from such cell line having the ATCC designation CRL-8220, whereby such mutant cell line will proliferate under conditions which will not sustain such cell line having the ATCC designation CRL-8220. Such mutant cell line may be deficient in hypoxanthine phosphoribosyl transferase; an example of such a cell line is the cell line having the ATCC designation CRL-8221. In preferred embodiments, the lymphocytes are human lymphocytes.

In another embodiment, cells of a hybrid cell line described above in turn are fused with lymphocytes which are preferably human lymphocytes. In still another embodiment, cells of a hybrid cell line described above are back-selected to restore the at least one characteristic which is different from the cell line having the ATCC designation CRL-8220, which at least one characteristic may be a hypoxanthine phosphoribosyl transferase deficiency. Mutants of the resulting back-selected cells then may be produced and fused with lymphocytes, preferably human lymphocytes.

The present invention further provides, in a process for producing a hybrid cell line by fusing lymphocytes with myeloma-derived cells to provide an anti-body-producing fused cell hybrid, the improvement which comprises employing human myeloma-derived cells from a perpetual human myeloma-derived cell line produced by treating cells from the human myeloma-derived cell line having the ATCC designation CRL-8220 with an agent to promote cell mutation and then selecting the treated cells with a suitable reagent to identify those treated cells having at least one characteristic which is different from the cell line having the ATCC designation CRL-8220, whereby such perpetual myeloma-derived cell line will proliferate under conditions which will not sustain such cell line having the ATCC designation CRL-8220. In one embodiment, such mutant cell line is deficient in hypoxanthine phosphoribosyl transferase. In another embodiment, such mutant cell line is the hypoxanthine phosphoribosyl transferase-deficient cell line having the ATCC designation CRL-8221. In yet another embodiment, the agent used to promote cell mutation is ethyl methane sulphonate and the selecting reagent is 6-thioguanine. In a preferred embodiment, the lymphocytes are human lymphocytes.

The present invention also provides a process for producing a hybrid cell line which comprises fusing cells of a mutant cell line with lymphocytes sensitized to an immunogen in the presence of an agent which promotes the fusion of such cells, which mutant cell line is derived from the cell line having the ATCC designation CRL-8220 and which is capable of hybridization with lymphocytes and which has at least one characteristic which is different from such cell line having the ATCC designation CRL-8220, whereby such mutant cell line will proliferate under conditions which will not sustain the cell line having the ATCC designation CRL-8220. Such mutant cell line may be a cell line which is deficient in hypoxanthine phosphoribosyl transferase, such as the cell line having the ATCC designation CRL-8221. In one embodiment, .the fusion-promoting agent is a polythylene glycol. In another embodiment, the lymphocytes are sensitized in vitro. In a preferred embodiment, the lymphocytes are human lymphocytes. In other embodiments, a hybrid cell line is produced by fusing cells of a hybrid cell line described above with lymphocytes sensitized to an immunogen in the presence of an agent which promotes the fusion of such cells.

The present invention further provides a process for the production of antibodies which comprises culturing cells of a hybrid cell line according to the present invention in a culture medium and then isolating from the medium the antibodies produced by such cells.

The present invention also provides, in a process for the transfection of lymphocytes, the improvement which comprises using the deoxyribonucleic acid from cells of the cell line having the ATCC designation CRL-8220.

The present invention further provides monoclonal antibodies produced by a hybrid cell line according to the present invention.

The human myeloma cell line having the ATCC designation CRL-8220 is useful in the production of hybrid cell lines, or hybridomas, which are capable of producing antibodies. Such hybrid cell lines in turn are useful as models for studying lymphocyte growth, regulation and differentiation; determining the location of genes expressed in lymphocytes; and examining the biochemical basis of neoplastic change. The monoclonal antibodies produced by such hybrid cell lines are of practical relevance to experimental and clinical immunology, molecular biology and cell biology, and are useful in clinical analyses, especially for immunoassays for minute quantities of haptens and antigens in body fluids and tissues.

In accordance with the present invention, a .continually proliferating (i.e. perpetual) human myeloma-derived cell line has been established. This cell line, designated AHM-I, originated from a culture of the peripheral blood lymphocytes of a 64-year old Caucasian male diagnosed as having multiple myeloma.

Analysis of the serum from this patient demonstrated elevated levels of IgG (11,200 mg/dl, the normal range being 660-1760 mg/dl) and slightly elevated levels of IgA (600 mg/dl, the normal range being 60-320 mg/dl). Serum electrophoresis indicated the presence of an M-component of the IgG type with lambda light chains. A bone marrow biopsy indicated a neoplastic invasion with cells which were described as having a plasmacytoid appearance.

Blood from this patient was fractionated on a Ficoll gradient. The interface cells were washed and suspended in RPMI 1640 medium containing 20% fetal calf serum, 2 mM glutamine and 50 µg/ml gentamicin. The resulting culture was incubated at 73°C in a humidified, 5% $CO_2$ atmosphere for six weeks. At the end of this time, clumps of cells were observed in the culture and appeared to be lymphoblastoid in nature. These cells were harvested and resuspended in 10 ml of fresh medium and incubated at 37°C for eight days. The resulting culture was harvested and contained a total of $7x10^5$ viable cells which were reseeded in fresh medium. Subsequent passages were made at 1:10 to 1:15 splits. The cell line, designated AHM-I, has been continually passaged for 12 months in culture. The doubling time appears to be between 24 and 48 hours. The cell line has been cloned by limiting dilution in the absence of feeder cells.

AHM-I appears to be a human lymphoblastoid cell line as determined by light and electron microscopy. The cells are not adherent or attachment dependent and grow in suspension culture. In culture, the cells have a tendency to aggregate into clumps of several thousand cells. The cell line grows from a seeding density of $5x10^4$ cells/ml to $2x10^6$ cells/ml in about six days.

Immunoprecipitation of expired AHM-I culture supernatants demonstrated that the cell line secretes an IgA with lambda light chains. $IgA_1$ heavy chains were detected on the surfaces of the cells. IgG has not been detected, either in the culture supernatants or on the cell surface.

Two human B lymphocyte markers, HLA-DR and B1 [Stashenko et al., J. Immunol., 125, 1678, (1980)] were detected on the surfaces of AHM-I cells. Additional evidence for the human origin of this cell line was obtained by enzyme analysis. AHM-I demonstrated the following human enzymes: nucleoside phosphorylase, glucose-6-phosphate dehydrogenase, malate dehydrogenase, mannose phosphate isomerase, peptidase B, aspartate aminotransferase and lactate dehydrogenase.

Hypoxanthine phosphoribosyl transferase-deficient (HPRT-deficient) mutants of AHM-I have been established by mutangenization with ethyl methane sulphonate in accordance with well-known procedures. Following mutagenization, HPRT-deficient variants were selected for by growing the cells in medium containing $7.5 \times 10^{-6}$ M 6-thioguanine which was gradually increased to $1 \times 10^{-4}$ M [Welsh, Nature, 256, 495, (1977)]. The HPRT-deficient mutants, contrary to AHM-I, will not grow in medium containing hypoxanthine ($1 \times 10^{-4}$ M), aminopterin ($4 \times 10^{-7}$ M) and thymidine ($1.6 \times 10^{-5}$ M) (HAT medium).

To date, between 50 and 100 HPRT-deficient mutants of AHM-I have been established. One such mutant, designated AHM-3D3, has been studied in detail. In general, AHM-3D3 will fuse with human peripheral blood lymphocytes, B lymphocytes isolated from human peripheral blood lymphocytes and lymphocytes from human lymph nodes.

Cell lines AHM-I and AHM-3D3 were deposited on 10 March 1983 with the American Type Culture Collection and assigned the ATCC accession numbers CRL-8220 and CRL-8221, respectively.

In general, the present invention encompasses all daughter lines of AHM-I, which is the cell line having the ATCC designation CRL-8220 as mentioned above, a cell line is considered to be a daughter line of AHM-I if it is derived therefrom, such as by mutation or hybridization. The term "derived" is used herein to embrace any cell line which includes the AHM-I cell line in its genealogy.

As noted above, a cell line may be derived from AHM-I by mutation. Such mutation may be the result of natural mutation or of the use of an agent which induces cell mutation in accordance with well-known procedures. Although various such agents are well known to those skilled in the art, a commonly used agent is ethyl methane sulphonate. Other agents, however, may be employed, such as ultra-violet radiation, methyl methane sulphonate, N-methyl-N'-nitro-N-nitrosoguanidine and ethyl nitrosourea; see , e.g., Jakoby and Pastan, Editors, "Methods in Enzymology. Cell Culture", Vol. 58, Academic Press, New York, 1979, p. 308. To aid in screening for cells having the desired characteristics, the cells are typically selected with a suitable reagent. For example, where the characteristic is hypoxanthine phosphoribosyl transferase deficiency, such suitable reagent is typically 6-thioguanine.

Apart from the capability of hybridization or fusing with lymphocytes, the characteristics of the cell line obtained from AHM-I by mutation are generally not critical. As a practical matter, however, such mutant cell line will have at least one characteristic which is different from AHM-I, whereby such mutant cell line will proliferate (i.e. is capable of continuous cultivation) under conditions which will not sustain AHM-I. This permits the ready separation of mutant cells from AHM-I cells. While a variety of such characteristics are known to those skilled in the art, an especially useful and well-known characteristic is hypoxanthine phosphoribosyl transferase deficiency. An example of a mutant cell line derived from AHM-I which which exhibits hypoxanthine phosphoribosyl transferase deficiency is AHM-3D3, the cell line having the ATCC designation CRL-8221.

A cell line also may be derived from AHM-I by hybridization which in general involves the fusion of cells from an AHM-I mutant cell line with lymphocytes in the presence of an agent which promotes fusion in accordance with well-known procedures. An especially well-suited fusion-promoting agent is polyethylene glycol having a molecular weight of about 1000. Other agents may, of course, be employed, such as electric field pulses [Teissie et al., Science, 216, 537, (1982)] and Sendai virus [Davidson, Experimental Cell Research, 55, 424, (1969)]. While cells from any mutant cell line may be employed, a mutant cell line having hypoxanthine phosphoribosyl transferase deficiency, such as AHM-3D3, is especially useful.

In general, the origin of the lymphocytes employed is not critical. Thus, such lymphocytes may be mouse, rat or human lymphocytes, or lymphocytes from some other species. For some applications, such as the production of monoclonal antibodies for use in an immunoassay for minute quantities of haptens or antigens in body fluids, mouse or rat lymphocytes may be preferred. In other instances, however, human lymphocytes may be desired. Indeed, the use of human lymphocytes is preferred.

The source of the lymphocytes also is not critical. Where the lymphocytes are human in origin, they may be peripheral blood lymphocytes, B lymphocytes isolated from peripheral blood lymphocytes or lymphocytes isolated .from lymph nodes or spleen.

Where the hybrid cell line is to be used for the production of monoclonal antibodies, the lymphocytes are typically sensitized to an immunogen. As used herein, the term "immunogen" includes both antigens and haptens. Such sensitization is carried out in accordance with procedures which are well known to those skilled in the art. As a practical matter, sensitization is typically carried out in vitro where the lymphocytes are of human origin. When non-human lymphocytes are employed, sensitization is typically accomplished in vivo.

In some cases, it may be desirable to back-select cells from a mutant cell line to restore the at least one characteristic which is different from AHN-I, e.g. hypoxanthine phosphoribosyl transferase deficiency, in accordance with known procedures. See, e.g., Shulman et al., Nature, 276, 269, (1978). Mutation of the resulting restored cells then may be carried out to produce a new mutant cell line which is capable of hybridization with lymphocytes and having at least one characteristic which is different from the restored cells, whereby the new mutant cell line will proliferate under conditions which will not sustain the restored cells. The new mutant cell line then may be fused with lymphocytes sensitized to an immunogen as discussed above.

Deoxyribonucleic acid from AHM-I cells may be used in the transfection of lymphocytes in accordance with known procedures. See, e.g., Jonak et al., Hybridoma, 2, 124 (1982); and Cooper, Science, 218, 801, (1982).

The present invention is further illustrated by the following Examples. Unless otherwise indicated, all temperatures are in degrees Celsius and all percentages are by weight.

EXAMPLE 1

Production of Hybrids Between AHM-3D3 and Human Peripheral Blood Lymphocytes

The million human peripheral blood lymphocytes isolated from a Ficoll gradient centrifugation of normal blood were mixed with $1 \times 10^7$ AHM-3D3 cells and the resulting cell mixture was pelleted by centrifugation at 800 X g for 10 minutes. The pellet was gently disrupted and warmed to 37°. One-half millilitre of 37% polyethylene glycol having a molecular weight of 1000 (Koch-Light Laboratories, Ltd.) was added to the disrupted pellet with stirring over a 50 second period. The resulting fusion mixture was diluted gradually with 5.0 ml of serum-free RPMI-1640 medium (RPMI-1640, 2 mM glutamine, 50 g/ml gentamicin, and $5 \times 10^{-5}$ M 2-mercaptoethanol) over a period of 2 minutes. A second 5.0 ml portion of medium was added over the next 1 minute period. The fusion mixture was centrifuged and the cells were washed twice with 50 ml portions of serum-free medium. The cells then were suspended at a density of $5 \times 10^6$ cells/ml in RPMI-1640 medium containing 20% fetal calf serum.

50 microlitres of the above cell suspension ($2.5 \times 10^5$ cells) was dispersed into each well of a 96-well microtiter plate which 24 hours previously had been seeded with $4 \times 10^3$ Balb/c mouse peritoneal exudate cells in 50 µl of RPMI-1640 containing 20% fetal calf serum per well. The plate was incubated overnight at 37°C in a humidified 5% $CO_2$ incubator. Following overnight incubation, 100 µl of serum-containing medium supplemented with 2X concentrated HAT ($2 \times 10^{-4}$ M hypoxanthine, $8 \times 10^{-7}$ M aminopterin and $3.2 \times 10^{-5}$ M thymidine) was added to each well and the plate incubated for an additional 5 days. The cultures then were fed every other day by removing some medium and replacing it with fresh 1X HAT medium.

3 to 4 weeks following fusion, some cultures contained actively growing cells, indicating successful fusions between AHM-3D3 and human lymphocytes. These fused cell products continued to proliferate in HAT-containing medium in which AHM-3D3 will not grow.

In addition to normal human peripheral blood lymphocytes, lymphocytes from human lymph nodes and populations of peripheral blood lymphocytes enriched for B lymphocytes have been fused to AHM-3D3. Table 1 below summarizes the results obtained from three such experiments.

## TABLE 1

Frequency of hybridoma formation obtained
by fusing AHM-3D3 with human lymphocytes
from various sources

| Fusion experiment No. | Source of lymphocytes | No. of cultures | No. of cultures positive for growth | No. of hybridomas per No. of input lymphocytes |
|---|---|---|---|---|
| 1 | Peripheral blood lympho-cytes | 72 | 5 | $1/1.8 \times 10^6$ |
| 2 | Lymphocytes from lymph nodes | 81 | 14 | $1/7.2 \times 10^5$ |
| 3 | B lymphocytes from peripheral blood | 13 | 6 | $1/2.5 \times 10^5$ |

Fusion studies to date indicate that fusion frequencies for AHM-3D3 with peripheral blood lymphocytes were 1 in $1-3 \times 10^6$ input lymphocytes. Such frequencies for lymph node cells were on the order of 1 in $7 \times 10^5 - 1 \times 10^6$ input lymphocytes. In fusions in which isolated B lymphocytes were used, the fusion frequency increased to 1 in $2-3 \times 10^5$ input lymphocytes, a frequency which compares very favourably to fusion frequencies obtainable in mouse fusion systems and which represents a distinct improvement over the most recently-reported human-human hybrid formation of 1 in $10^6 - 10^8$ input lymphocytes [Edwards et al., European Journal of Immunology, 12, 641, 1982].

EXAMPLE 2

Immunoglobulin production by AHM 3D3 hybridomas

10 of the hybridomas resulting from Example 1 were tested for the synthesis of an immunoglobulin different from that produced by the parent cell line, AHM-3D3. Cells from each hybridoma cell line were cultured at a concentration of $1\times10^6$ cells/ml in leucine-free RPMI-1640 medium containing 5 μCi/ml $^{14}$C-labelled leucine for 48 hours. Each culture supenatant then was isolated and subjected to immunoprecipitation with various immunoglobulin isotype-specific antisera in accordance with well known procedures. The resulting immunoprecipates were washed and dissociated by adding pH 6.8 Tris-HCl buffer containing 10% glycerol, 2% sodium dodecyl sulphate and 5% 2-mercaptoethanol and boiling for 2 minutes.

The samples then were subjected to electrophoresis on a 5-20% polyacrylamide gradient gel in 0.25 M Tris buffer at pH 8.3 containing 0.192 M glycine and 0.1% sodium dodecyl sulphate. Following electrophoresis, the gels were fixed by staining with 0.125% Coomassic Blue in an aqueous solution containing 50% methanol and 10% glacial acetic acid. The gels then were soaked in dimethyl sulphoxide to remove water. The dimethyl sulphoxide was replaced with a 22.2% solution (weight/volume) of 2,5-diphenyloxazole (PPO) in dimethyl sulphoxide. Following incubation for 2 hours at ambient temperature, the PPO solution was removed and replaced with distilled water for 1 hour. The gels then were dried and exposed to Kodak XAR-5 x-ray film over a Dupont Lightening Plus intensifying screen overnight at -70°.

The electrophoresis patterns from the gels clearly indicate that nine of the ten hybridoma cell lines secrete IgM molecules with kappa light chains. The tenth hybridoma secretes an IgA molecule which has a kappa light chain. AHM-3D3, however, secretes an IgA molecule with a lambda light chain.

CLAIMS

1.      A cell line characterised in that it is a substantially pure human myeloma-derived cell line having the ATCC designation CRL-8220.

2.      A cell line which is derived from a cell line as claimed in claim 1.

3.      A cell line as claimed in claim 2 which is derived by mutation or hybridization.

4.      A mutant cell line as claimed in claim 3 which is capable of hybridization with lymphocytes, which has at least one characteristic which is different from the cell line having the ATCC designation CRL-8220 and which is capable of proliferation under conditions which will not sustain such cell line.

5.      A mutant cell line as claimed in claim 4 which is deficient in hypoxanthine phosphoribosyl transferase.

6.      A mutant cell line as claimed in claim 5 having the ATCC designation CRL-8221.

7.      A hybrid cell line as claimed in claim 3 which is obtainable from cells of a mutant cell line as claimed in any of claims 4 to 6 by fusion thereof with lymphocytes sensitized to an immunogen.

8.      A hybrid cell line which is obtainable by the fusion of cells of a hybrid cell line as claimed in claim 7 with lymphocytes sensitized to an immunogen.

9.      A hybrid cell line as claimed in claim 7 or claim 8 wherein the lymphocytes are human lymophcytes.

10.    A cell line which is obtainable from cells of a hybrid cell line as claimed in claim 7 by back-selecting the cells to restore the at least one characteristic which is different from the cell line having the ATCC designation CRL-8220.

11.    A mutant cell line which has at least one characteristic which is different from a cell line as claimed in claim 10, which is capable of hybridization with lymphocytes and which is capable of proliferation under conditions which will not sustain such cell line.

12.    A hybrid cell line which is obtainable by the fusion of cells of a mutant cell line as claimed in claim 11 with lymphocytes sensitized to an immunogen.

13.    A process for the production of an antibody-producing hybrid cell line characterised in that it comprises fusing lymphocytes with myeloma-derived cells, there being used human myeloma-derived cells from a perpetual human myeloma-derived cell line obtainable by treating cells from the human myeloma-derived cell line having the ATCC designation CRL-8220 as claimed in claim 1 or cells from a cell line as claimed in claim 2 with an agent to promote cell mutation and then selecting the treated cells with a reagent to identify those treated cells having at least one characteristic which is different from such cell line, the perpetual human myeloma-derived cell line being capable of proliferation under conditions which will not sustain such cell line.

14.    A process as claimed in claim 13 wherein the agent is ethyl methane sulphonate and the reagent is 6-thioguanine.

15.    A process as claimed in claim 13 or claim 14 wherein the mutant cell line is deficient in hypoxanthine phosphoribosyl transferase and/or has the ATCC designation CRL-8221.

16.    A process for the production of a hybrid cell line characterised in that it comprises fusing cells of a mutant cell line as claimed in any of claims 3 to 6 with lymphocytes sensitized to an immunogen in the presence of an agent which promotes the fusion of such cells.

17.    A process as claimed in claim 16 wherein the agent is a polyethylene glycol.

18.    A process as claimed in any of claims 13 to 17 wherein the lymphocytes are human lymphocytes.

19.    A process as claimed in any of claims 16 to 18 wherein the lymphocytes are sensitized _in vitro_.

20.    A process for the production of a hybrid cell line characterised in that it comprises fusing cells of a hybrid cell line as claimed in claim 7 with lymphocytes sensitized to an immunogen in the presence of an agent which promotes the fusion of such cells.

21.    A process for the production of antibodies characterised in that it comprises culturing cells of a hybrid cell line as claimed in any of claims 3, 7, 8 or 12 in a culture medium and then isolating therefrom the antibodies produced.

22.    A process for the transfection of lymphocytes characterised in that it comprises using the deoxyribonucleic acid from cells of a cell line as claimed in any of claims 1 to 3.

European Patent
Office

**EUROPEAN SEARCH REPORT**

| | **DOCUMENTS CONSIDERED TO BE RELEVANT** | | EP 84302307.8 |
|---|---|---|---|
| Category | Citation of document with indication, where appropriate, of relevant passages | Relevant to claim | CLASSIFICATION OF THE APPLICATION (Int. Cl. ³) |
| A,D | GB - A - 2 086 937 (THE WISTAR INSTITUTE)<br><br>* Claims 1,4,5,8,9,12,14,17; page 1, line 125 - page 2, line 1; page 2, lines 26, 55-59, 99, 100 *<br><br>-- | 1-5,7-21 | C 12 N   5/00<br>C 12 N 15/00<br>C 12 N   5/02<br>A 61 K 39/395 |
| A,D | PROCEEDINGS OF THE NATIONAL ACA-DEMY OF SCIENCES OF THE UNITED STATES OF AMERICA, vol. 77, no.9, September 1980<br><br>L. OLSSON et al. "Human-human hybridomas producing monoclonal antibodies of predefined antigenic specifity" pages 5429-5431<br><br>* Pages 5429, 5430 *<br><br>-- | 1-5,7-13,15-18,20,21 | |
| A,D | EUROPEAN JOURNAL OF IMMUNOLOGY, vol. 12, no. 8, Augsut 1982, Weinheim<br><br>P.A.W. EDWARDS et al. "A human-human hybridoma system based on a fast-growing mutant of the ARH-77 plasma cell leukemia-derived line" pages 641-648<br><br>* Page 641 - page 642, column 2, line 39 *<br><br>-- | 13,15-21 | **TECHNICAL FIELDS SEARCHED (Int. Cl. ³)**<br><br>C 12 N<br>A 61 K |
| A | EP - A1 - 0 044 722 (LELAND STANFORD JUNIOR UNIVERSITY)<br><br>* Claims 1,9 *<br><br>-- | 1-5 | |

The present search report has been drawn up for all claims

| Place of search | Date of completion of the search | Examiner |
|---|---|---|
| VIENNA | 13-07-1984 | FARNIOK |

0124301

European Patent Office

**EUROPEAN SEARCH REPORT**

Application number

EP 84302307.8

| **DOCUMENTS CONSIDERED TO BE RELEVANT** | | | CLASSIFICATION OF THE APPLICATION (Int. Cl.) |
|---|---|---|---|
| Category | Citation of document with indication, where appropriate, of relevant passages | Relevant to claim | |
| A,D | SCIENCE, vol. 216, no. 4549, May 1982, Washington, D.C.<br><br>A. KARPAS et al. "Human Myeloma Cell Line Carrying a Philadelphia Chromosome"<br>pages 997-999<br><br>* Totality *<br><br>---- | 1,2 | |
| | | | **TECHNICAL FIELDS SEARCHED (Int. Cl.)** |

EPO Form 1503.2  06.78